# EUROPEAN PATENT APPLICATION

(11) **EP 3 498 200 A1**
(43) Date of publication of application: **19.06.2019**
(21) Application number: 17838505.0
(22) Date of filing: 12.07.2017
(51) Int. Cl.: A61B 17/32

(54) **ULTRASONIC SCALPEL HANDLE**

(30) Priority: 08.08.2016 CN 201620845590 U
(71) Applicant: Jiangsu SMTP Technology Co., Ltd., Zhangjiagang, Jiangsu 215634 (CN)
(72) Inventor: LUO, Zhuojing, Zhangjiagang Jiangsu 215634 (CN); ZHAN, Songtao, Zhangjiagang Jiangsu 215634 (CN); CAO, Qun, Zhangjiagang Jiangsu 215634 (CN)
(74) Representative: Daub, Thomas
(86) International application number: PCT/CN2017/092569
(87) International publication number: WO 2018/028375

(57) **Abstract**

Disclosed is an ultrasonic scalpel handle, comprising a tool bit mounting portion (1), a transducer mounting portion (2) connected to a rear end of the tool bit mounting portion (1), and a handheld portion (3) connected to a rear end of the transducer mounting portion (2). The ultrasonic scalpel handle has two bending structures consisting of a front bending portion (4) and a rear bending portion (5), and the front bending portion (4) and the rear bending portion (5) have opposite bending directions. The ultrasonic scalpel handle of the invention is simple in structure, convenient for fabrication, flexible in operation and of wide range of applications. When the ultrasonic scalpel of the present invention is used in operation, a position for an operator to hold the ultrasonic scalpel is changed, thereby removing a gripping portion which may block a line of sight outside a surgical view, so that a doctor's surgical view is clearer, especially in the field of minimally invasive surgery requiring fine observation, the ultrasonic scalpel handle of the present invention can exert its advantages. The ultrasonic scalpel handle of the present invention has a wider range of applications, shorter operation time, higher surgical safety and less patient suffering.

## Description

### TECHNICAL FIELD

The present disclosure relates to the field of medical instruments and devices, and more particularly to an ultrasonic scalpel handle.

### BACKGROUND OF THE INVENTION

In modern clinical medicine, ultrasonic surgical equipment has been gradually applied in various fields such as orthopedics, neurosurgery, burns, ophthalmology, etc. A surgeon holds an ultrasonic scalpel and performs a surgical operation. Except that an ultrasonic hemostatic scalpel uses a clamping type handle, other types of ultrasonic scalpels typically use straight or bending handles, which has had a wide applications in open surgery. Bending handles can also better meet general requirements for viewing.

In the current minimally invasive surgery, due to the limitation of holding ways, the straight or curved handle each has a problem of obscuring the doctor's line of sight, which greatly limits the applications of the ultrasonic scalpel, resulting in that this advanced technology cannot be used to a wider range in the field of surgery.

### SUMMARY OF THE INVENTION

In order to solve the above problems in the prior art, the present disclosure provides an ultrasonic scalpel handle having a double-bend structure, the ultrasonic scalpel handle comprises a tool bit mounting portion, a transducer mounting portion connected to a rear end of the tool bit mounting portion, and a handheld portion connected to a rear end of the transducer mounting portion, wherein the ultrasonic scalpel handle has two bending structures consisting of a front bending portion and a rear bending portion, respectively, and the front bending portion and the rear bending portion have opposite bending directions.

In the ultrasonic scalpel handle of the present invention, preferably, the front bending portion is formed on the transducer mounting portion, and a rear straight segment of the transducer mounting portion having formed with the front bending portion forms an angle with the handheld portion so as to form the rear bending portion.

In the ultrasonic scalpel handle of the present invention, preferably, the rear straight segment of the transducer mounting portion is connected with the handheld portion by a hinge capable of adjusting a bending angle, and the hinge has a locking mechanism.

In the ultrasonic scalpel handle of the present invention, preferably, the front bending portion is formed on the transducer mounting portion, and the rear bending portion is formed on the handheld portion.

In the ultrasonic scalpel handle of the present invention, preferably, the front bending portion is formed on the tool bit mounting portion, and the rear bending portion is formed on the handheld portion.

In the ultrasonic scalpel handle of the present invention, preferably, a bent tool bit is attached to the tool bit mounting portion to form the front bending portion, and the rear bending portion is formed on the handheld portion.

In the ultrasonic scalpel handle of the present invention, preferably, the rear bending portion formed on the handheld portion has a hinge for adjusting a bending angle, and the hinge has a locking mechanism.

In the ultrasonic scalpel handle of the present invention, preferably, a bent tool bit is attached to the tool bit mounting portion to form the front bending portion, and the rear bending portion is formed on the transducer mounting portion.

In the ultrasonic scalpel handle of the present invention, preferably, a length of the handheld portion is freely adjustable.

In the ultrasonic scalpel handle of the present invention, preferably, the handheld portion and the transducer mounting portion are of an integral structure or a separate structure.

The ultrasonic scalpel handle of the invention is simple in structure, convenient for fabrication, flexible in operation and of wide range of applications. When the ultrasonic scalpel of the present invention is used in operation, a position for an operator to hold the ultrasonic scalpel is changed, thereby removing a gripping portion which may block a line of sight, to the outside of a surgical view, so that a doctor's surgical view is clearer, especially in the field of minimally invasive surgery requiring fine observation, the ultrasonic scalpel handle of the present invention can exert its advantages. The ultrasonic scalpel handle of the present invention has a wider range of applications, shorter operation time, higher surgical safety and less patient suffering. The handheld portion of the handle can be produced separately and easily, and the angle and length of the handheld portion are adjustable, making the operation more flexible.

### BRIEF DESCRIPTION OF THE DRAWINGS

In order to clearly describe the technical solutions of the embodiments of the present invention or the prior art, a brief description will be made for the drawings to describe the embodiments. Apparently, the drawings provided only show some embodiments of the present invention. For those skilled in the art, other drawings may be obtained according to the disclosed drawings without inventive labor.
Fig. 1 is a schematic perspective view of an ultrasonic scalpel handle according to a first embodiment of the present invention;
Fig. 2 is a schematic cross-sectional view of the ultrasonic scalpel handle according to the first embodiment of the present invention;
Fig. 3 is a schematic perspective view of an ultrasonic scalpel handle according to a second embodiment of the present invention;
Fig. 4 is a schematic cross-sectional view of the ultrasonic scalpel handle according to the second embodiment of the present invention;
Fig. 5 is a schematic perspective view of an ultrasonic scalpel handle according to a third embodiment of the present invention; and
Fig. 6 is a schematic cross-sectional view of the ultrasonic scalpel handle according to the third embodiment of the present invention.

### List of Reference Numerals:

1∼tool bit mounting portion; 2∼transducer mounting portion; 3∼handheld portion; 4∼front bending portion; 5∼rear bending portion; 6∼hinge.

### DETAILED DESCRIPTION OF THE INVENTION

Exemplary embodiments of the present invention will be described hereinafter clearly and completely with reference to the attached drawings. Apparently, the embodiments described herein are only portions of embodiments of the invention, rather than all embodiments of the invention. It is intended that all other embodiments obtained by those skilled in the art according to the disclosed embodiments without inventive labor are all within the scope of the present invention.

In the description of the present invention, it is to be noted that the terms of "center", "upper", "lower", "left", "right", "vertical", "horizontal", "internal", "external" and the like simply indicate orientational or positional relationship based on the accompanying drawings and are used only for the purpose of facilitating and simplifying the description of the invention, rather than specifying or implying that any devices or elements indicated must have a certain orientation, be configured or operate in a certain orientation. Therefore, these terms will not be interpreted as limiting the present invention. Further, the terms of "first", "second" and "third" and the like are only used for describing purpose, rather than being interpreted as specifying or implying relative importance.

In the description of the present disclosure, it is to be noted that, unless otherwise specified or defined clearly, the term of "attach", "connect to", "connect with", "couple" and the like should be interpreted broadly. For example, they may refer to fixed connection, or detachable connection, or integral connection; they may refer to mechanical connection, or electrical connection; they may refer to direct connection, or indirect connection through an intermediate agent, or internal communication between two components. For those skilled in the art, the specific meaning of these terms in the present disclosure may be understood in combination with specific situations or contexts.

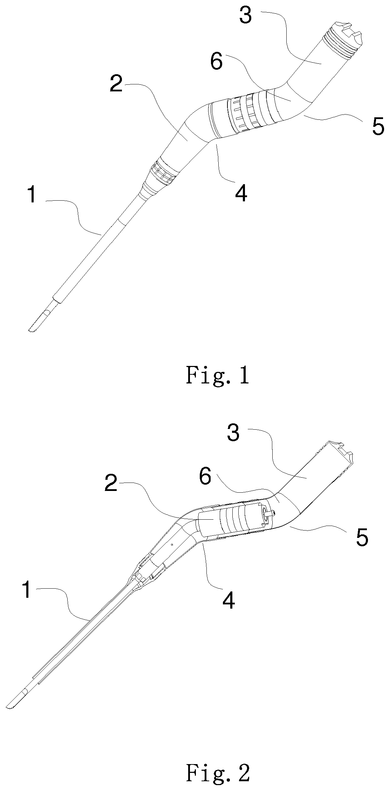

Fig. 1 is a schematic perspective view of an ultrasonic scalpel handle according to a first embodiment of the present invention. Fig. 2 is a schematic cross-sectional view of the ultrasonic scalpel handle according to the first embodiment of the present invention. As shown in Figs.1 and 2, the ultrasonic scalpel handle according to the first embodiment of the present invention includes a tool bit mounting portion 1, a transducer mounting portion 2 connected to a rear end of the tool bit mounting portion 1, and a handheld portion 3 connected to a rear end of the transducer mounting portion 2. The ultrasonic scalpel handle has two bending structures, i.e., a front bending portion 4 and a rear bending portion 5, respectively, and the front bending portion 4 and the rear bending portion 5 has opposite bending directions. An ultrasonic tool bit and a water injection sleeve may be attached to the tool bit mounting portion 1. The front bending portion 4 and the rear bending portion 5 together form a double-bend structure that is similar to a bend structure of a human leg. During a surgery, a doctor can hold a vicinity of the rear bending portion 5 with single hand for an operation, or hold the transducer mounting portion 2 and the handheld portion 3 with two hands for an operation. The tool bit mounting portion 1 below the front bending portion 4 is completely exposed to the doctor's field of view, so that the doctor can clearly observe the condition of a site for a surgical operation. On that basis, an amplification apparatus can also be used to assist in a minimally invasive surgery, which greatly expands the range of applications for the ultrasonic scalpel handle, improves safety of the operation, reduces burdens on the doctor, and reduces sufferings of a patient.

Further referring to Figs. 1 and 2, in the ultrasonic scalpel handle according to the first embodiment of the present invention, the front bending portion 4 is formed on the transducer mounting portion 2, and a rear straight segment of the transducer mounting portion 2 having formed with the front bending portion 4 forms an angle with the handheld portion 3 so as to form a rear bending portion 5. Optionally, the handheld portion 3 and the rear straight segment of the transducer mounting portion 2 may also be connected with each other by a hinge 6 capable of adjusting a bending angle, and a locking member (not shown) may be provided on the hinge 6.

Further, in the ultrasonic scalpel handle according to the first embodiment of the present invention, alternatively, the front bending portion 4 may be formed on the transducer mounting portion 2, and the rear bending portion 5 may be formed on the handheld portion 3. The handheld portion 3 may be provided with a hinge 6 for adjusting a bending angle, and the hinge 6 may be provided with a locking member (not shown). The main function of the handheld portion 3 is to facilitate an operator to hold the ultrasonic scalpel handle, and there are no electronic devices therein, so that the bending angle of the rear bending portion 5 of the handheld portion 3 can be fixed or adjustable. A length of the handheld portion 3 can be fixed or be freely adjusted, which is convenient for use. As described above, the rear bending portion 5 on the handheld portion 3 has a hinge 6 for adjusting the bending angle, so that an operator can adjust the hinge 6 into an appropriate angle according to a surgical need, and then lock the locking member to fix the hinge 6 at an angle required in the surgery.

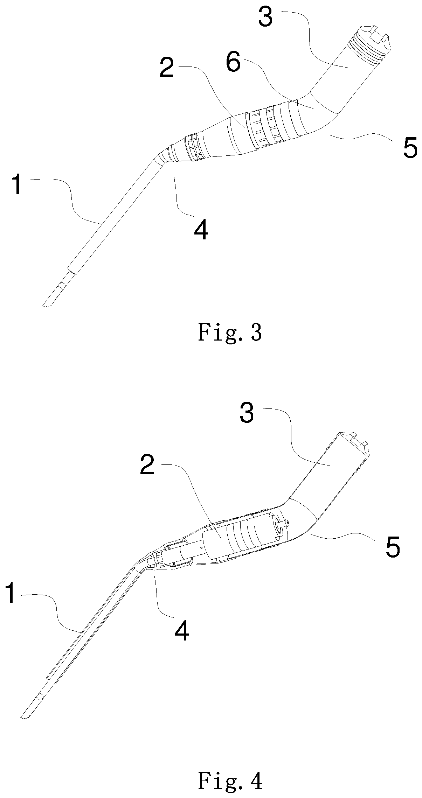

Fig. 3 is a schematic perspective view of an ultrasonic scalpel handle according to a second embodiment of the present invention, and Fig. 4 is a schematic cross-sectional view of the ultrasonic scalpel handle according to the second embodiment of the present invention. As shown in Figs. 3 and 4, in the ultrasonic scalpel handle according to the second embodiment of the present invention, a front bending portion 4 is formed on the tool bit mounting portion 1, and a rear bending portion 5 is formed on the handheld portion 3. In the second embodiment, since there is no bending formed on the transducer mounting portion 2, the transducer mounting portion 2 has a straight structure, so that a linear ultrasonic transducer can be mounted, which is advantageous for fabricating the ultrasonic transducer and improving the performance of the ultrasonic transducer. Optionally, the rear bending portion 5 on the handheld portion 3 may be provided with a hinge 6 for adjusting a bending angle, and the hinge 6 may be provided with a locking member (not shown). During a surgery, once an operator adjusts the hinge 6 into an appropriate angle according to a surgical need and locks the locking member, the hinge 6 can be fixed at a desired angle to achieve the best surgical effect.

Further, as a modification of the second embodiment, a bent tool bit may be attached to the tool bit mounting portion 1 to form the front bending portion 4, and the rear bending portion bend 5 may be formed on the handheld portion 3. Also, since there is no bending formed on the transducer mounting portion 2, a linear ultrasonic transducer can be mounted, which is advantageous for fabricating the ultrasonic transducer and for improving the performance of the ultrasonic transducer. Optionally, the rear bending portion 5 on the handheld portion 3 may be provided with a hinge 6 for adjusting a bending angle, and the hinge 6 may be provided with a locking member (not shown). During a surgery, once an operator adjusts the hinge 6 into an appropriate angle according to a surgical need and locks the locking member, the hinge 6 can be fixed at a desired angle to achieve the best surgical effect.

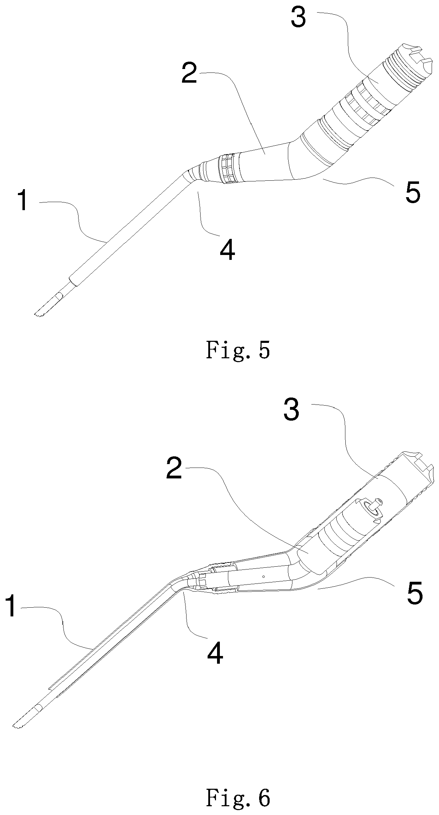

Fig .5 is a schematic perspective view of an ultrasonic scalpel handle according to a third embodiment of the present invention, and Fig. 6 is a schematic cross-sectional view of the ultrasonic scalpel handle according to the third embodiment of the present invention. As shown in Figs. 5 and 6, in the ultrasonic scalpel handle according to the third embodiment of the present invention, a bent tool bit may be attached to the tool bit mounting portion 1 to form the front bending portion, and the rear bending portion is formed on the transducer mounting portion 2. In this case, since the main function of the handheld portion 3 is to facilitate an operator to hold the ultrasonic scalpel handle, there are no electronic devices therein, so that a bending angle of the rear bending portion of the handheld portion 3 can be fixed or adjusted. A length of the handheld portion 3 can be fixed or freely adjusted to facilitate the operation during a surgery.

In the above embodiments of the invention, the handheld portion 3 and the transducer mounting portion 2 may be integrally formed to form an integral structure. The integral structure of the handheld portion 3 and the transducer mounting portion 2 is more simple and compact and has a longer service life.

In the above embodiments of the present invention, the handheld portion 3 and the transducer mounting portion 2 may also have a separate structure. With the separate structure, the handheld portion 3 and the transducer mounting portion 2 can be separately produced, which is easy for fabrication and replacement.

Compared with the prior art, the embodiments of the present invention have the following advantages: the ultrasonic scalpel handle of the invention is simple in structure, convenient for fabrication, flexible in operation and of wide range of applications. When the ultrasonic scalpel of the present invention is used in operation, a position for an operator to hold the ultrasonic scalpel is changed, thereby removing a gripping portion which may block a line of sight outside a surgical view, so that a doctor's surgical view is clearer, especially in the field of minimally invasive surgery requiring fine observation, the ultrasonic scalpel handle of the present invention can exert its advantages.

It should be noted that the above embodiments are only used to describe the concept of the present invention, rather than limiting the present invention. Although detailed descriptions of the invention are made with reference to the above embodiments, it would be appreciated by those skilled in the art that various changes or modifications to the above embodiments may be made or equivalent substitutions to portion of or all features in those embodiments may be made. Such changes, modifications or substitutions will not make the spirit of the relevant solutions depart from the scope of the present invention.

## Claims

1. An ultrasonic scalpel handle, comprising a tool bit mounting portion, a transducer mounting portion connected to a rear end of the tool bit mounting portion, and a handheld portion connected to a rear end of the transducer mounting portion, wherein
the ultrasonic scalpel handle has two bending structures consisting of a front bending portion and a rear bending portion, and the front bending portion and the rear bending portion have opposite bending directions.

2. The ultrasonic scalpel handle of claim 1, wherein
the front bending portion is formed on the transducer mounting portion, and a rear straight segment of the transducer mounting portion having formed with the front bending portion forms an angle with the handheld portion so as to form the rear bending portion.

3. The ultrasonic scalpel handle of claim 2, wherein
the rear straight segment of the transducer mounting portion is connected with the handheld portion by a hinge capable of adjusting a bending angle, and the hinge has a locking mechanism.

4. The ultrasonic scalpel handle of claim 1, wherein
the front bending portion is formed on the transducer mounting portion, and the rear bending portion is formed on the handheld portion.

5. The ultrasonic scalpel handle of claim 1, wherein
the front bending portion is formed on the tool bit mounting portion, and the rear bending portion is formed on the handheld portion.

6. The ultrasonic scalpel handle of claim 1, wherein
a bent tool bit is attached to the tool bit mounting portion to form the front bending portion, and the rear bending portion is formed on the handheld portion.

7. The ultrasonic scalpel handle of any one of claims 4 to 6, wherein
the rear bending portion formed on the handheld portion has a hinge for adjusting a bending angle, and the hinge has a locking mechanism.

8. The ultrasonic scalpel handle of claim 1, wherein
a bent tool bit is attached to the tool bit mounting portion to form the front bending portion, and the rear bending portion is formed on the transducer mounting portion.

9. The ultrasonic scalpel handle of any one of claims 1 to 6 and 8, wherein
a length of the handheld portion is freely adjustable.

10. The ultrasonic scalpel handle of any one of claims 1 to 6 and 8, wherein
the handheld portion and the transducer mounting portion are of an integral structure or a separate structure.
